# EUROPÄISCHE PATENTANMELDUNG

(11) **EP 2 397 474 A1**
(43) Veröffentlichungstag der Anmeldung: **21.12.2011**
(21) Anmeldenummer: 10166244.3
(22) Anmeldetag: 17.06.2010
(51) Int. Cl.: C07D 317/34

(54) **2-Oxo-1,3-dioxolan-4-carbonsäureester, ihre Herstellung und Verwendung**

(71) Anmelder: Construction Research & Technology GmbH, 83308 Trostberg (DE)
(72) Erfinder: Mecfel-Marczewski, Joanna, 83352, Altenmarkt an der Alz (DE); Walther, Burkhard, 84518, Garching (DE); Mezger, Jochen, 84518, Garching an der Alz (DE); Kierat, Radoslaw, 83352, Altenmarkt (DE); Staudhamer, Rosita, 83547, Babensham (DE)

(57) **Zusammenfassung**

Vorgeschlagen werden neue 2-Oxo-1,3-dioxolan-4-carbonsäureester der Formel 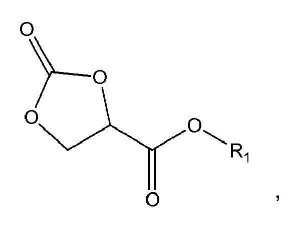 bei denen R₁ vorzugsweise Me oder Et oder ein n-wertiger Rest sein kann, der mit maximal n-1 weiteren 2-Oxo-1,3-dioxolan-4-carboxylgruppen substituiert sein kann, ein Verfahren zu ihrer Herstellung mittels Carboxylierung der entsprechenden Epoxide, ein Verfahren zu ihrer Umesterung sowie ihre Verwendung zur Herstellung von Hydroxyurethanen und als Endgruppen zum Blockieren von Aminen.

## Beschreibung

Die vorliegende Erfindung betrifft neue 2-Oxo-1,3-dioxolan-4-carbonsäureester der Formel 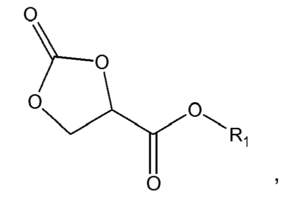 bei denen R₁ vorzugsweise Me oder Et oder ein n-wertiger Rest sein kann, der mit maximal n-1 weiteren 2-Oxo-1,3-dioxolan-4-carboxylgruppen substituiert sein kann, Verfahren zu ihrer Herstellung, Verfahren zu ihrer Umesterung sowie ihre Verwendung zur Herstellung von Hydroxyurethanen und als Endgruppen zum Blockieren von Aminen.

Strukturell ähnliche Verbindungen sind im Stand der Technik bereits bekannt. So beschreibt WO2004/003001 A1 Verbindungen der allgemeinen Formel (I) 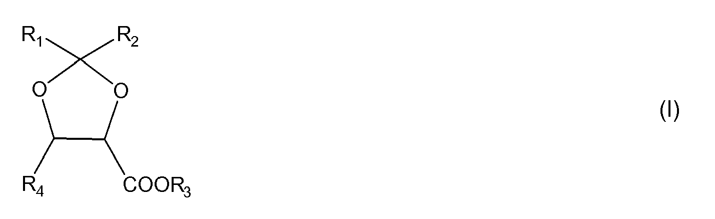 wobei R₁ und R₂ voneinander unabhängige Reste, R₁+R₂ = O oder CR₁+R₂ eine 3-6-gliedrige Cycloalkylgruppe sein können. R₄ kann Wasserstoff, geradkettiges oder verzweigtes C₁₋₈-Alkyl, C₅₋₁₂-Cycloalkyl oder C₆₋₁₅-Aryl sein. R₃ kann geradkettiges oder verzweigtes C₁₋₈-Alkyl oder C₆₋₁₅-Aryl sein. Allgemein beschreibt WO2004/003001 die enzymatische Racematentrennung der Enantiomeren vom Typ (I), ohne jedoch eine Synthese für diese Verbindungen anzugeben.

EP 1941946 A1 beschreibt die Verwendung eines Carbonitrid-Katalysators u.a. zur Herstellung bestimmter disubstituierter organischer Carbonate. Dabei kann es sich auch um Verbindungen der allgemeinen Formel (II) handeln, 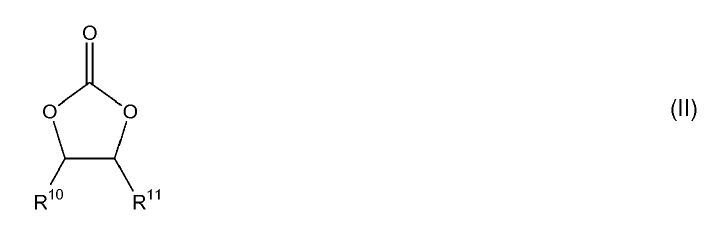 wobei R¹⁰ und R¹¹ unabhängig voneinander ausgewählte, optionale Substituenten sind. Mögliche Bedeutungen der Substituenten sind Alkyl, Aryl, Herteroaryl und Estergruppen CO₂A, wobei A wiederum Alkyl oder Aryl sein kann, z.B. geradkettiges oder verzweigtes C₁₋₆-Alkyl, bevorzugt C₁₋₃-Alkyl und besonders bevorzugt Methyl oder Ethyl. Es werden indessen keine Synthesen für 2-Oxo-1,3-dioxolan-Systeme angegeben.

Aus JP 2006-003433 A ist eine Dichtmasse für Flüssigkristalldisplayelemente bekannt, welche eine Verbindung der allgemeinen Formel (III) umfasst, 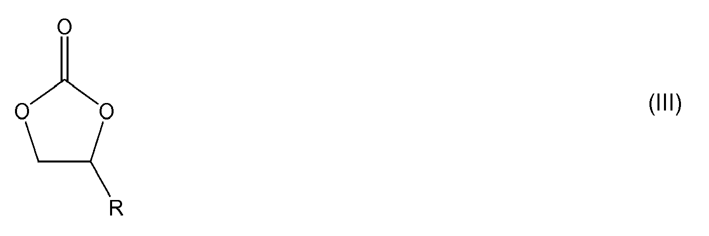 wobei R für H, eine Hydroxylgruppe, eine Cyanogruppe, eine Carbonsäuregruppe, einen ggf. substituierten aromatischen Ring, eine geradkettige, verzweigte oder cyclische Alkylgruppe, eine Acylgruppe oder eine Estergruppe steht. Es wird indessen nicht gesagt, in welche Richtung die Estergruppe zeigt und welchen weiteren Rest sie trägt. Es wird auch keine konkrete Synthese für diese 2-Oxo-1,3-dioxolan-Systeme angegeben.

In EP 0001088 A1 werden u.a. 2-Oxo-1,3-dioxolane der allgemeinen Formel (IV) beschrieben, wobei R = H oder CH₃ sein kann.

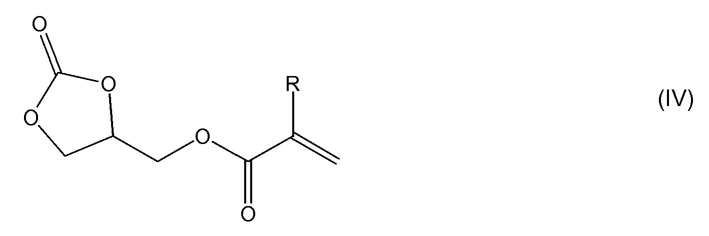

Polyurethane auf Basis von Polyisocyanaten gehören zum Stand der Technik. Diese werden beispielsweise als Klebstoffe, Dichtstoffe, Vergussmassen, als Korrosionsschutz und für Beschichtungen verwendet. Vorteilhaft sind die hohe Säure-, Laugen-und Chemikalienbeständigkeit der so erhaltenen gehärteten Zusammensetzungen. Allerdings sind monomere niedermolekulare (Poly)Isocyanatverbindungen toxikologisch bedenklich, insbesondere wenn diese leichtflüchtig sind oder migrieren.

Polyurethan-Systeme können auch ausgehend von cyclischen Carbonatverbindungen erhalten werden, welche toxikologisch unbedenklich sind. So findet Glycerincarbonat (4-(Hydroxymethyl)-2-oxo-1,3-dioxolan) beispielsweise Anwendung in Kosmetika. Cyclische Carbonatverbindungen reagieren mit Aminen zu Hydroxyurethanen.

Einfache cyclische Carbonate wie z.B. 4-Methyl-2-oxo-1,3-dioxolan oder besagtes 4-(Hydroxymethyl)-2-oxo-1,3-dioxolan sind indessen nicht besonders reaktionsfähig. Es wurden Studien durchgeführt, vergl. H. Tomita, F. Sanda, T. Endo, Journal of Polymer Science: Part A: Polymer Chemistry, Vol. 39, 3678-3685 (2001), wonach die Reaktionsfähigkeit der in 4-Position mit der Gruppe R substituierten 2-Oxo-1,3-dioxolane mit Aminen in der Reihenfolge: R = Me < R = H < R = Ph < R = CH₂OPh << R = CF₃ ansteigt. Leider sind solche fluorierten Verbindungen schwer zugänglich, teuer und (z.B. im Brandfall) potentiell toxisch. Zudem sind niedermolekulare, monomere 2-Oxo-1,3-dioxolane nicht als Bindemittel geeignet. Vielmehr sind reaktionsfähige funktionelle Gruppen beispielsweise in 4-Position erforderlich um höhermolekulare, multifunktionelle Bindemittel herzustellen, die dann zur Polyurethanbildung mit Aminen umgesetzt werden können. Auch die industrielle Zugänglichkeit dieser 2-Oxo-1,3-dioxolane spielt eine wichtige Rolle.

Die der vorliegenden Erfindung zu Grunde liegende Aufgabe war es, zumindest einige der Nachteile des vorstehend geschilderten Standes der Technik im Wesentlichen zu vermeiden. Insbesondere sollte ein 2-Oxo-1,3-dioxolan-System zur Verfügung gestellt werden, das unbedenklich, gut zugänglich und mit Aminen hochreaktiv ist und zumindest eine weitere bzw. weitere reaktionsfähige funktionelle Gruppe(n) trägt.

Diese Aufgabe wurde mit den Merkmalen der unabhängigen Ansprüche gelöst. Die abhängigen Ansprüche beziehen sich auf bevorzugte Ausführungsformen.

Gegenstand der vorliegenden Erfindung sind 2-Oxo-1,3-dioxolan-4-carbonsäureester der Formel (V), 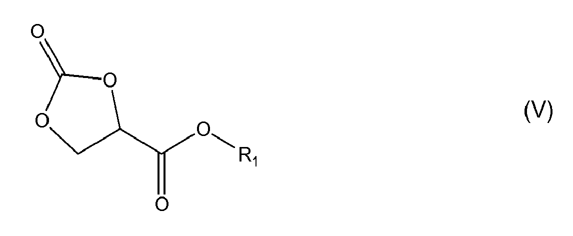 welche dadurch gekennzeichnet sind, dass R₁ aus geradkettigen oder verzweigten aliphatischen Gruppen, Arylgruppen, Aralkylgruppen sowie Alkarylgruppen ausgewählt ist.

In diesen 2-Oxo-1,3-dioxolan-4-carbonsäureestern kann R₁ beispielsweise aus geradkettigen oder verzweigten Alkylgruppen, insbesondere Methyl, Ethyl, n-Propyl, iso-Propyl, n-Butyl, sec-Butyl, tert-Butyl, n-Pentyl, neo-Pentyl, n-Hexyl, 2-Ethyl-n-hexyl, sowie aus Cyclohexyl, Phenyl und Benzyl ausgewählt sein.

Insbesondere handelt es sich bei dem erfindungsgemäßen 2-Oxo-1,3-dioxolan-4-carbonsäureester um 4-Methoxycarbonyl-2-oxo-1,3-dioxolan bzw. 4-Ethoxycarbonyl-2-oxo-1,3-dioxolan.

Es ist allerdings ebenfalls möglich, dass R₁ ein durch Abstraktion der OH -Gruppen eines n-wertigen Polyols abgeleiteter n-wertiger Rest ist, der mit maximal n-1 weiteren 2-Oxo-1,3-dioxolan-4-carboxylatgruppen der allgemeinen Formel (VI) 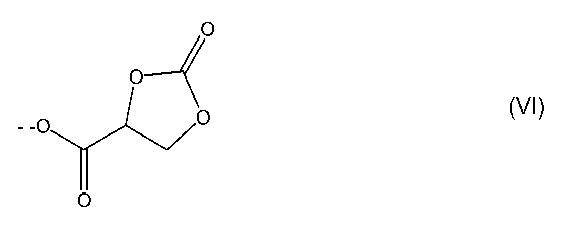 substituiert sein kann. Wenn weniger als n-1 weitere 2-Oxo-1,3-dioxolan-4-carboxylatgruppen anwesend sind, ist R₁ zusätzlich mit der stöchiometrisch notwendigen Anzahl an OH -Gruppen substituiert.

In diesen höhermolekularen 2-Oxo-1,3-dioxolan-4-carbonsäureestern kann das n-wertige Polyol beispielsweise C₂₋₄-(Poly)Oxyalkylengruppen umfassen, d.h. von Ethylenoxid, Propylenoxid und/oder Butylenoxid abgeleitete Gruppen mit einer oder mehreren Oxyalkylen-Wiederholungseinheiten. Vorzugsweise ist n = 2 bis 5. Beispiele solcher höhermolekularen, multifunktionellen, als Bindemittel geeigneten Verbindungen werden weiter unten diskutiert.

Ein weiterer Gegenstand der vorliegenden Erfindung ist in der Herstellung der erfindungsgemäßen, niedermolekularen, monomeren 2-Oxo-1,3-dioxolan-4-carbonsäureester zu sehen. Diese 2-Oxo-1,3-dioxolan-4-carbonsäureester können beispielsweise hergestellt werden, indem die entsprechenden Epoxide der Formel (VII), wobei R₁ die angegebene Bedeutung aufweist, mit CO₂ umgesetzt werden.

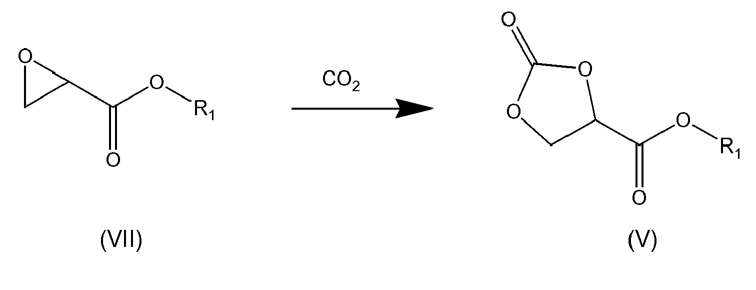

Epoxide der Formel (VII) sind im Stand der Technik wohlbekannte, kommerziell erhältliche Verbindungen, die beispielsweise mittels Epoxidierung der entsprechenden Acrylsäureester (VIII) hergestellt werden können; für R₁ = Me vergl. z.B. Organic Syntheses, Vol. 83, S. 162 (2006):

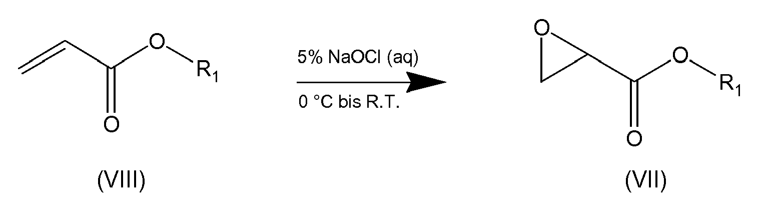

Auch alternative Synthesen sind bekannt; für R₁ = Et vergl. z.B. Organic Syntheses, Coll. Vol. 10, S. 401 (2004); Vol. 75, S. 37 (1998).

Die Temperatur der oben erwähnten Umsetzung mit CO₂ kann in weiten Bereichen variiert werden. Sie sollte zweckmäßig im Bereich von 15 °C bis 150 °C, vorzugsweise im Bereich von 30 °C bis 100 °C, liegen. Die Umsetzung kann in offenen Apparaturen bei Umgebungsdruck (ca. 1 bar) durchgeführt werden, beispielsweise mittels Durchleitens von gasförmigem CO₂ durch eine geeignete Reaktionslösung. Die Umsetzung kann aber auch in geschlossenen Systemen bei erhöhtem Druck erfolgen, beispielsweise bei einem Druck von 1 bar bis 75 bar, vorzugsweise bei einem Druck von 1 bar bis 30 bar.

Die Umsetzung mit CO₂ kann ohne Lösungsmittel erfolgen, da die Ausgangsstoffe und die Produkte in der Regel flüssig sind. Es hat sich jedoch als zweckmäßig erwiesen, die Umsetzung in polaren aprotischen Lösungsmitteln durchzuführen. Eine nicht abschließende Aufzählung geeigneter Lösungsmittel umfasst Tetrabutylmethyleter, Acetonitril, Aceton, Tetrahydrofuran, Dimethylcarbonat, Toluol, Xylol, N-Methyl-2-pyrrolidon, N-Ethyl-2-pyrrolidon sowie Mischungen davon.

Die Umsetzung mit CO₂ kann in der Regel ohne Katalysator durchgeführt werden. Zweckmäßig wird jedoch in Gegenwart eines Katalysators gearbeitet, der aus Metallhalogeniden und Halogensalzen organischer Stickstoffverbindungen sowie Mischungen davon, ausgewählt ist.

Wie weiter oben festgestellt wurde, sind niedermolekulare, monomere 2-Oxo-1,3-dioxolan-4-carbonsäureester nicht als Bindemittel geeignet. Die COOR₁ -Gruppe weist indessen den Vorteil auf, dass sie für weitere Umsetzungen zur Verfügung steht. So können höhermolekulare, multifunktionelle Vertreter der erfindungsgemäßen Verbindung der allgemeinen Formel (V) mittels Umesterung hergestellt werden. Demgemäß ist ein Verfahren zur Herstellung dieser höhermolekularen, multifunktionellen Ester als ein weiterer Gegenstand der vorliegenden Erfindung zu werten, wobei ein niedermolekularer, monomerer 2-Oxo-1,3-dioxolan-4-carbonsäureester der allgemeinen Formel (V) mit einem n-wertigen Polyol umgeestert wird.

In dem besagten Verfahren wird die Umesterung in Gegenwart eines enzymatischen Katalysators oder eines sauren Kationenaustauschers durchgeführt. Eine der Schwierigkeiten, die mit dieser Umesterungsreaktion verbunden war, bestand darin, Katalysatoren zu finden, die zwar die Umesterung an der -COOR₁ -Gruppe katalysieren, nicht aber zu Angriffen auf die -O-COO- Gruppe führen. Die oben angegebenen Katalysatoren umgehen diese Schwierigkeiten. Als besonders geeignet haben sich Novozym^{®} 435 der Fa. Novozymes A/S, eine immobilisierte Lipase, und die H⁺-Form von Amberlite^{®} 200 der Fa. Rohm & Haas Company, d.h. eines stark sauren Kationenaustauschers, erwiesen.

Das Polyol und der niedermolekulare 2-Oxo-1,3-dioxolan-4-carbonsäureester werden vorzugsweise in stöchiometrischen Anteilen eingesetzt, wobei der Umsatz der Umesterung vorzugsweise über 80 %, bezogen auf den eingesetzten niedermolekularen 2-Oxo-1,3-dioxolan-4-carbonsäureester, liegen sollte. Die Reaktionstemperatur liegt im Bereich von 50 bis 100 °C, wobei Novozym^{®} 435 bei etwa 50 bis 80 °C und Amberlite^{®} 200 bei ca. 100 °C eingesetzt werden sollte. Die Umesterung mit Novozym^{®} 435 kann zweckmäßig ohne Lösungsmittel durchgeführt werden, die Umesterung mit Amberlite^{®} 200 wird zweckmäßig in einem geeigneten Lösungsmittel durchgeführt. Die Reaktion wird zweckmäßig solange durchgeführt, bis im Wesentlichen die berechnete Menge an R₁-OH abdestilliert ist.

Geeignete Polyole sind beispielsweise Diole, insbesondere Glykole, Triole und Tetraole wie z.B. 1,4-Butandiol, Neopentylglykol (2,2-Dimethylolpropan), 1,1,1-Trimethylolpropan, Pentaerythrit und Tetramethylolmethan. Die besagten Polyole können auch mit C₂₋₄-Alkylenoxiden, insbesondere Ethylenoxid und Propylenoxid, modifiziert sein. Allgemein sind alle Polyole einsetzbar, die auch für die Herstellung herkömmlicher Polyurethane verwendbar sind.

Alternativ ist es möglich, niedermolekulare Acrylsäureester zunächst mit den besagten Polyolen umzuestern, anschließend zu epoxidieren und dann mit CO₂ zu carboxylieren. Man erhält so Verbindungen, die ebenfalls unter die allgemeine Formel (V) fallen.

Ein weiterer Gegenstand der Erfindung ist die Verwendung der 2-Oxo-1,3-dioxolan-4-carbonsäureester zur Herstellung von Hydroxyurethanen. Die erfindungsgemäßen cyclischen Carbonatverbindungen reagieren mit Aminen zu Hydroxyurethanen.

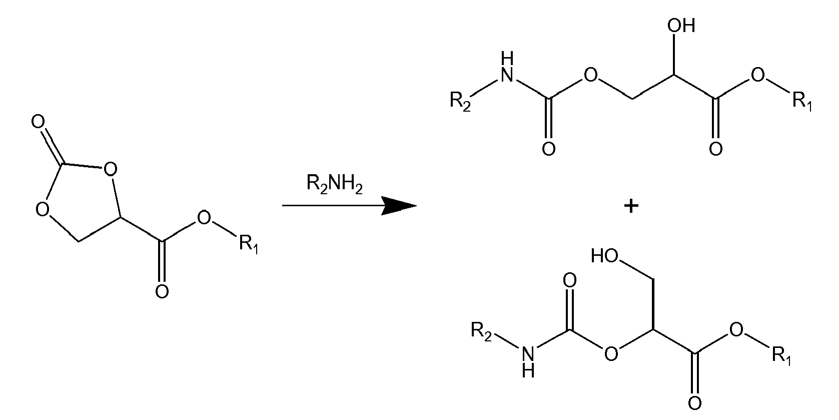

Hierbei sind grundsätzlich zwei unterschiedliche Hydroxyurethane möglich, nämlich Hydroxyurethane mit primären bzw. sekundären Hydroxylgruppen. Es hat sich indessen gezeigt, dass die elektronenziehende COOR₁ -Gruppe die Reaktion im Wesentlichen in Richtung der Hydroxyurethane mit sekundären Hydroxylgruppen lenkt, da beim Angriff des nucleophilen Stickstoffatoms die negative Ladung am Sauerstoffatom, das der COOR₁ -Gruppe näher liegt, besser stabilisiert wird. Hydroxyurethane mit sekundären Hydroxylgruppen weisen den zusätzlichen Vorteil auf, dass die Rückreaktion erschwert ist. Theoretisch denkbar wäre auch ein Angriff des Amins an der Estergruppe; es konnte aber analytisch gezeigt werden, dass das Amin im Wesentlichen nur die 2-Oxo-1,3-dioxolangruppe angreift.

Als Amine kommen primäre und sekundäre Amine mit Alkylgruppen, Arylgruppen, Aralkylgruppen sowie Alkarylgruppen als Reste in Frage. Primäre Amine reagieren schneller als sekundäre Amine; aliphatische Amine reagieren schneller als aromatische Amine. Hinsichtlich der relativen Reaktivitäten verschiedener Amine vergleiche C. Diakoumakos, D. Kotzev, Non-Isocyanate-Based Polyurethanes Derived upon the Reaction of Amines with Cyclocarbonate Resins, Macromol. Symp., 216, 37-46 (2004), insbesondere Schema 4 auf S. 45. Alle dort genannten Amine eignen sich auch zur Durchführung der vorliegenden Erfindung. Auch höhermolekulare Amine wie z.B. Jeffamine^{®} der Fa. Huntsman Corp. und Polyetheramine der BASF SE kommen in Frage.

Wie weiter unten anhand von Beispielen gezeigt wird, sind die erfindungsgemäßen 2-Oxo-1,3-dioxolan-4-carbonsäureester gegenüber Aminen wesentlich reaktiver als beispielsweise die vergleichbaren Verbindungen Glycerincarbonat (4-(Hydroxymethyl)-2-oxo-1,3-dioxolan) und Propylencarbonat (4-Methyl-2-oxo-1,3-dioxolan). Dies gilt sowohl für die niedermolekularen als auch für die höhermolekularen Vertreter. Die Reaktivität des erfindungsgemäßen 4-Methoxycarbonyl-2-Oxo-1,3-dioxolans gegenüber Aminen dürfte in der Größenordnung des in "H. Tomita, F. Sanda, T. Endo, Journal of Polymer Science: Part A: Polymer Chemistry, Vol. 39, 3678-3685 (2001)" untersuchten 4-Trifluormethyl-2-oxo-1,3-dioxolans liegen, ohne jedoch die oben beschriebenen Nachteile der CF₃ -Gruppe aufzuweisen.

Ein zusätzlicher Vorteil von Poly-Hydroxyurethansystemen liegt in der höheren Hydrophilie dieser Systeme, was auf die enthaltenen OH -Gruppen zurückzuführen ist. Diese OH -Gruppen stehen prinzipiell auch zur Vernetzung mit Polyisocyanaten zur Verfügung, wobei die erfindungsgemäß möglichen, isocyanatfreien Systeme aufgrund ihrer geringeren Toxizität indessen bevorzugt werden.

Außerdem kann es bei der Erzeugung von Poly-Hydroxyurethansystemen, die auf 2-Oxo-1,3-dioxolanen beruhen, auch bei der Anwesenheit von Feuchtigkeit nicht zur Blasenbildung durch entstehendes CO₂ kommen. Dadurch werden weitgehend poren-und blasenfreie Beschichtungen möglich, was bei klassischen Polyurethansystemen mitunter problematisch ist. Des Weiteren ist auch die thermische Stabilität solcher Poly-Hydroxyurethansysteme höher als die Stabilität klassischer Polyurethansysteme.

Zudem können die niedermolekularen 2-Oxo-1,3-dioxolan-4-carbonsäureester dazu verwendet werden, als Endgruppen (sog. "end caps") Amine zu blockieren, was einen weiteren Gegenstand der vorliegenden Erfindung darstellt. Dies ist auch im Hinblick auf herkömmliche, aminvernetzte Polyurethan-Systeme von Interesse, da ein Aminüberschuss zu Verfärbungen führen kann, während ein Isocyanatüberschuss toxikologisch bedenklich ist.

Die vorliegende Erfindung wird nun anhand der nachfolgenden Beispiele unter Bezug auf die beigefügten Zeichnungen näher erläutert. Hierbei zeigen:
- Fig. 1: den zeitlichen Verlauf der Umsetzung verschiedener 2-Oxo-1,3-dioxolane mit Ethanolamin,
- Fig. 2: den zeitlichen Verlauf der Umsetzung verschiedener 2-Oxo-1,3-dioxolane mit Benzylamin,
- Fig. 3: den zeitlichen Verlauf der Umsetzung verschiedener 2-Oxo-1,3-dioxolane mit Isophorondiamin,
- Fig. 4: den zeitlichen Verlauf der Umsetzung verschiedener 2-Oxo-1,3-dioxolane mit Jeffamin^{®} D 400,
- Fig. 5: den zeitlichen Verlauf der Umsetzung verschiedener auf 2-Oxo-1,3-dioxolanen basierenden Bindemittel mit n-Butylamin.

### Beispiel 1: Herstellung von 4-Methoxycarbonyl-2-oxo-1,3-dioxolan

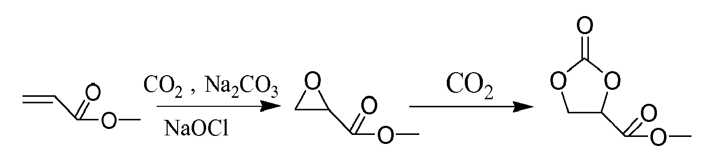

In einem 1000 ml Dreihalskolben wurden 80 g Natriumcarbonat in 200 ml destilliertem Wasser gelöst. Die Lösung wurde auf 10 °C abgekühlt. Anschließend wurden 58,5 g Methylacrylat zugegeben und nach ca. 10 Minuten ebenfalls bei 10 °C 400 ml einer 7 %-igen wässrigen Natriumhypochloritlösung eingerührt. Daraufhin wurde sofort intensiv mit CO₂ gespült. Man ließ die Temperatur auf Raumtemperatur steigen. Der Kolben wurde noch 1 h bei ca. 25 bis 30 °C intensiv mit CO₂ gespült, wobei die Temperatur durch gelegentliches Kühlen mit einem Eisbad im angegebenen Bereich gehalten wurde. Der entstandene weiße Feststoff wurde über eine Nutsche abfiltriert. Das Filtrat wurde mit 4 x 90 ml Dichlormethan extrahiert. Die vereinigte organische Phase wurde mit Natriumsulfat getrocknet und abfiltriert. Das Filtrat wurde am Rotationsverdampfer abrotiert. Es wurde Epoxypropionsäuremethylester in 50 bis 60 %-iger Ausbeute und einer Reinheit von 97% erhalten.

20 g des Epoxypropionsäuremethylesters wurden mit 20 g tert-Butyl-methylether und 1 g Tetrabutylammoniumbromid gemischt. Die homogene Mischung wurde in einen 100 ml Druckreaktor überführt und 4 Tage bei 40 °C und 20 bar CO₂-Druck carboxyliert. Nach der Carboxylierung wurde ein Zweiphasensystem erhalten; die obere Phase bestand aus tert-Butyl-methylether, die untere Phase aus 4-Methoxycarbonyl-2-oxo-1,3-dioxolan (Reinheit 94 % (GC), Ausbeute 94 %).

Das Produkt wurde wie folgt charakterisiert: ¹H NMR (500 MHz, CDCl₃) 6: 3,82 (3H, s, CH₃), 4,50 (1H, dd, J = 5,5, 9,0, CH₂), 4,66 (1H, dd, J = 9,0, 9.0, CH₂), 5,09 (1H, dd, J = 9,0, 5,5, CH); ¹³C NMR (125 MHz, CDCl₃) 6: 53,81 (CH₃), 67,00 (CH₂), 72,34 (CH), 153,97 (-O-CO-O-), 167,42 (-CO-O-); IR (neat): 1812 cm⁻¹, (-O-CO-O-), 1742 cm⁻¹ (-CO-O-).

### Beispiel 2: Herstellung von 4-Methoxycarbonyl-2-oxo-1,3-dioxolan

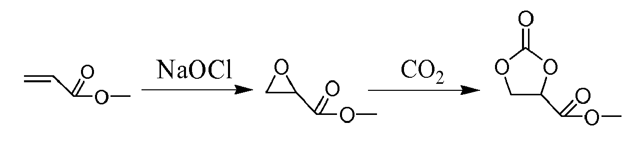

940 ml einer 7 %-igen wässrigen Natriumhypochloritlösung wurden in einem 2000 ml Dreihalskolben vorgelegt. Die Lösung wurde mit Hilfe eines Eis-Salz-Wasserbades auf 0 °C abgekühlt. Anschließend wurden 58,5 g Methylacrylat zugegeben und 30 Minuten bei 0 °C gehalten. Danach wurde die Kältemischung entfernt und ca. 1,5 h weitergerührt, so dass sich die Mischung von selbst erwärmte (65 - 70 °C). Es entstand eine farblose, trübe Lösung. Danach wurde die Lösung auf Raumtemperatur abgekühlt und mit 4 x 150 ml Dichlormethan extrahiert. Die vereinigte organische Phase wurde mit Magnesiumsulfat getrocknet und abfiltriert. Das Filtrat wurde am Rotationsverdampfer abrotiert. Es wurde Epoxypropionsäuremethylester in 70 bis 80 %-iger Ausbeute und einer Reinheit von 97% erhalten. Die weitere Umsetzung zum 4-Methoxycarbonyl-2-oxo-1,3-dioxolan verlief wie in Beispiel 1 beschrieben.

### Beispiel 3: Herstellung von 4-Methoxycarbonyl-2-oxo-1,3-dioxolan

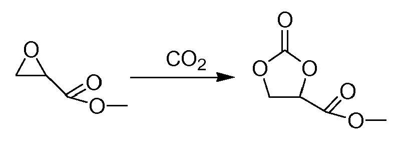

20 g Epoxypropionsäuremethylester wurden mit 20 g Acetonitril, 1,5 g Benzyltrimethylamoniumchlorid und 1,5 g ZnBr₂ gemischt. Die homogene Mischung wurde in einen 100 ml Druckreaktor überführt und 6 Tage bei 25 °C und 30 bar CO₂-Druck carboxyliert. Nach der Carboxylierung wurde mit 100 g Acetonitril verdünnt. Die die Mischung wurde mit Aluminiumoxid und Aktivkohle gereinigt. Danach wurde das Acetonitril abdestilliert. Man erhielt 4-Methoxycarbonyl-2-oxo-1,3-dioxolan (Reinheit 72 % (GC), Ausbeute 65 %).

### Beispiel 4: Herstellung von 4-Methoxycarbonyl-2-oxo-1,3-dioxolan

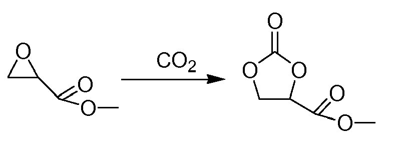

20 g Epoxypropionsäuremethylester wurden mit 20 g tert-Butyl-methylether, 1,5 g Tetrabutylammoniumbromid und 1,5 g Kaliumiodid gemischt. Die homogene Mischung wurde in einen 100 ml Druckreaktor überführt und 6 Tage bei 50 °C und 30 bar CO₂-Druck carboxyliert. Nach der Carboxylierung wurde ein Zweiphasensystem erhalten; die obere Phase bestand aus tert-Butyl-methylether, die untere Phase aus 4-Methoxycarbonyl-2-oxo-1,3-dioxolan (Reinheit 83 % (GC), Ausbeute 79 %).

### Beispiel 5: Herstellung Bindemittel 1

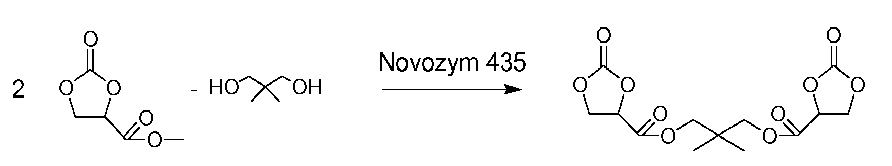

0,2 mol 4-Methoxycarbonyl-2-oxo-1,3-dioxolan ("GECA") wurden mit 0,1 mol Neopentylglykol (Sigma-Aldrich) gemischt. Dazu wurden 5 Gew.-% (bezogen auf GECA) Novozym^{®} 435 (Novozymes A/S) gegeben. Die Mischung wurde gerührt und auf 55 bis 60 °C erhitzt. Nach 72 h waren 0,2 mol Methanol abdestilliert und die Reaktion war beendet.

### Beispiel 6: Herstellung Bindemittel 2

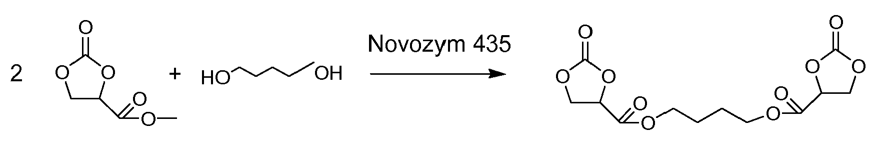

0,2 mol 4-Methoxycarbonyl-2-oxo-1,3-dioxolan ("GECA") wurden mit 0,1 mol 1,4-Butandiol (Sigma-Aldrich) gemischt. Dazu wurden 5 Gew.-% (bezogen auf GECA) Novozym^{®} 435 (Novozymes A/S) gegeben. Die Mischung wurde gerührt und auf 55 bis 60 °C erhitzt. Nach 72 h waren 0,2 mol Methanol abdestilliert und die Reaktion war beendet.

### Beispiel 7: Herstellung Bindemittel 3

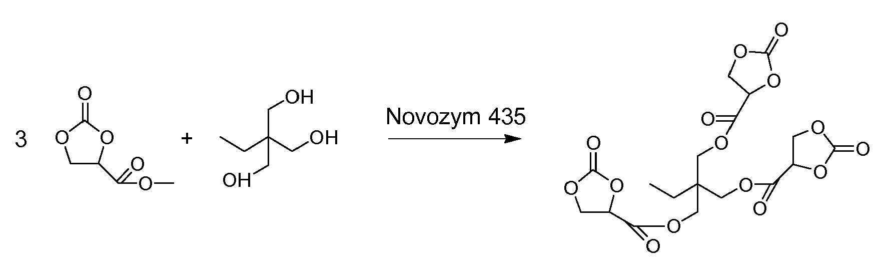

0,3 mol 4-Methoxycarbonyl-2-oxo-1,3-dioxolan ("GECA") wurden mit 0,1 mol 1,1,1-Trimetylolpropan (Sigma-Aldrich) gemischt. Dazu wurden 5 Gew.-% (bezogen auf GECA) Novozym^{®} 435 (Novozymes A/S) gegeben. Die Mischung wurde gerührt und auf 55 bis 60 °C erhitzt. Nach 72 h waren 0,3 mol Methanol abdestilliert und die Reaktion war beendet.

### Beispiel 8: Herstellung Bindemittel 4

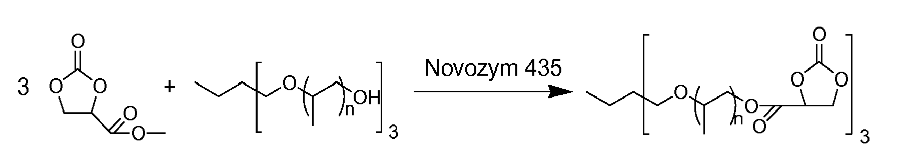

0,3 mol 4-Methoxycarbonyl-2-oxo-1,3-dioxolan ("GECA") wurden mit 0,1 mol 1,1,1-Trimethylolpropan-propoxylat (Sigma-Aldrich, mittleres Molekulargewicht (Mₙ) ca. 308) gemischt. Dazu wurden 5 Gew.-% (bezogen auf GECA) Novozym^{®} 435 (Novozymes A/S) gegeben. Die Mischung wurde gerührt und auf 55 bis 60 °C erhitzt. Nach 72 h waren 0,3 mol Methanol abdestilliert und die Reaktion war beendet.

### Beispiel 9: Herstellung Bindemittel 1

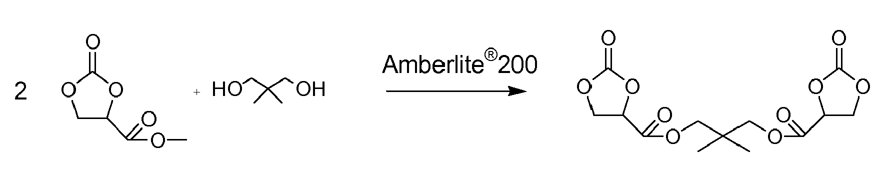

0,2 mol 4-Methoxycarbonyl-2-oxo-1,3-dioxolan ("GECA") wurden mit 0,1 mol Neopentylglykol (Sigma-Aldrich) und 200ml Cyclohexan gemischt. Dazu wurden 5 Gew.-% (bezogen auf GECA) Amberlite^{®} 200 (Fluka) zugegeben. Die Mischung wurde am Wasserabscheider bei 100°C gerührt. Nach 5 Stunden waren 0,2 mol Methanol abgeschieden und die Reaktion war beendet.

### Beispiel 10: Herstellung Bindemittel 3

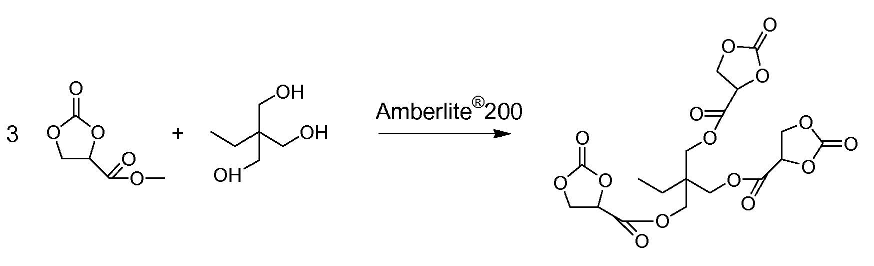

0,3 mol 4-Methoxycarbonyl-2-oxo-1,3-dioxolan ("GECA") wurden mit 0,1 mol 1,1,1-Trimetylolpropan (Sigma-Aldrich) gemischt. Dazu wurden 5 Gew.-% (bezogen auf GECA) Amberlite^{®} 200 (Fluka) zugegeben. Die Mischung wurde am Wasserabscheider bei 100°C gerührt. Nach 5 Stunden waren 0,3 mol Methanol abgeschieden und die Reaktion war beendet.

### Beispiel 11: Reaktion von 2-Oxo-1,3-dioxolanen mit Ethanolamin

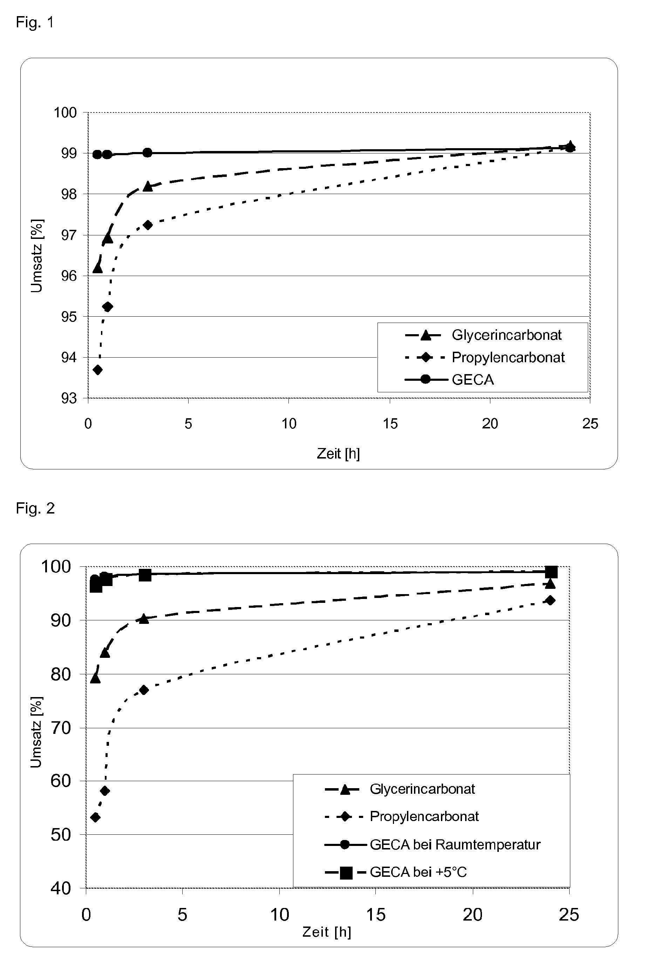

0,1 mol Ethanolamin wurden mit 0,1 mol 4-Methoxycarbonyl-2-oxo-1,3-dioxolan ("GE-CA") gemischt und bei Raumtemperatur gerührt. Nach 0,5 h, 1 h, 3 h und 24 h wurde die Aminzahl mittels Titration bestimmt und daraus der Umsatz berechnet. Diese Prozedur wurde auch mit 4-Methl-2-oxo-1,3-dioxolan ("Propylencarbonat") und 4-(Hydroxymethyl)-2-oxo-1,3-dioxolan ("Glycerincarbonat") durchgeführt. Die Ergebnisse sind in Fig. 1 graphisch wiedergegeben und zeigen die hohe Reaktivität des 4-Methoxycarbonyl-2-oxo-1,3-dioxolans.

### Beispiel 12: Reaktion von 2-Oxo-1,3-dioxolanen mit Benzylamin

Beispiel 11 wurde unter Verwendung von Benzylamin statt Ethanolamin wiederholt. 4-Methoxycarbonyl-2-oxo-1,3-dioxolan ("GECA") wurde sowohl bei Raumtemperatur als auch bei +5 °C untersucht, während 4-Methl-2-oxo-1,3-dioxolan ("Propylencarbonat") und 4-(Hydroxymethyl)-2-oxo-1,3-dioxolan ("Glycerincarbonat") lediglich bei Raumtemperatur getestet wurden. Die Ergebnisse sind in Fig. 2 graphisch wiedergegeben und zeigen eindrucksvoll die überragende Reaktivität des 4-Methoxycarbonyl-2-oxo-1,3-dioxolans (selbst bei + 5 °C).

### Beispiel 13: Reaktion von 2-Oxo-1,3-dioxolanen mit Isophorondiamin

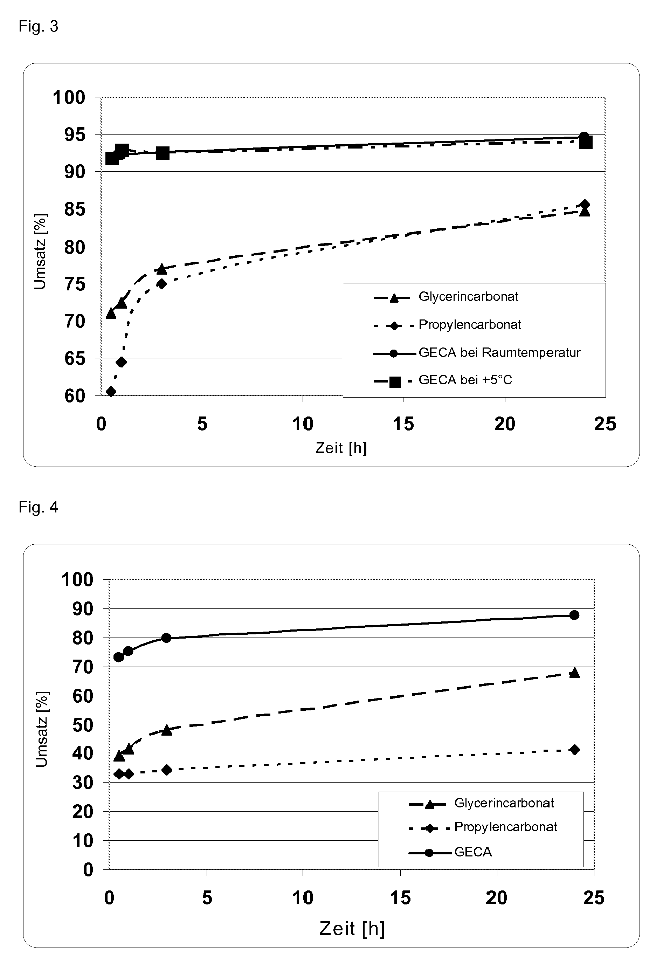

0,1 mol Isophorondiamin wurde mit 0,2 mol 4-Methoxycarbonyl-2-oxo-1,3-dioxolan ("GECA") gemischt und bei Raumtemperatur gerührt. Nach 0,5h, 1 h, 3h und 24h wurde die Aminzahl mittels Titration bestimmt und daraus der Umsatz berechnet. Diese Prozedur wurde auch mit 4-Methoxycarbonyl-2-oxo-1,3-dioxolan ("GECA") bei +5 °C und mit 4-Methl-2-oxo-1,3-dioxolan ("Propylencarbonat") und 4-(Hydroxymethyl)-2-oxo-1,3-dioxolan ("Glycerincarbonat") bei Raumtemperatur durchgeführt. Die Ergebnisse sind in Fig. 3 graphisch wiedergegeben und zeigen eindrucksvoll die hervorragende Reaktivität des 4-Methoxycarbonyl-2-oxo-1,3-dioxolans (selbst bei + 5 °C).

### Beispiel 14: Reaktion von 2-Oxo-1,3-dioxolanen mit Jeffamin® D 400

Beispiel 13 wurde unter Verwendung von Jeffamin^{®} D 400 wiederholt. Da die Reaktion mit hochmolekularen Aminen allgemein langsamer als mit niedermolekularen Aminen verlief, wurde auch das 4-Methoxycarbonyl-2-oxo-1,3-dioxolan (GECA) nur bei Raumtemperatur untersucht. Die Ergebnisse sind in Fig. 4 graphisch wiedergegeben und zeigen auch im vorliegenden Fall die hohe Reaktivität des 4-Methoxycarbonyl-2-oxo-1,3-dioxolans.

### Beispiel 15: Reaktion von Bindemittel 3 im Vergleich zum Stand der Technik

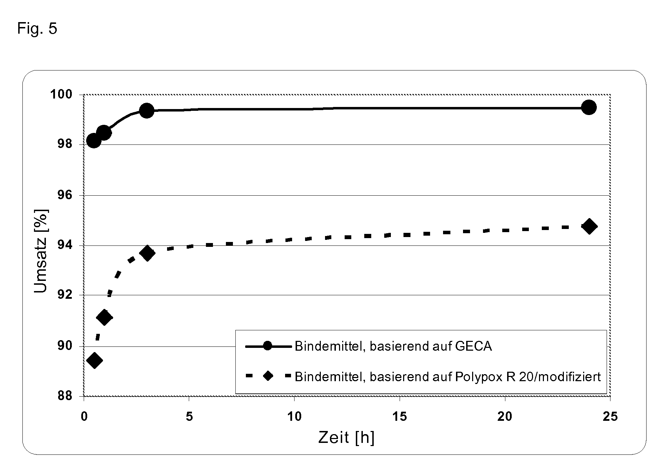

0,1 mol des Bindemittels 3 aus Beispiel 7 bzw. 10 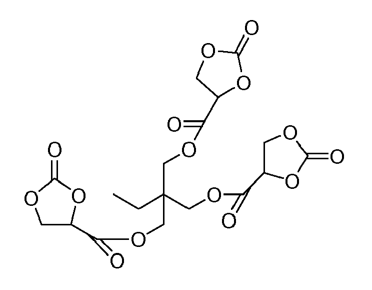 wurden mit 0,3 mol n-Butylamin gemischt und bei Raumtemperatur gerührt. Nach 0,5 h, 1 h, 3 h und 24 h wurde die Aminzahl mittels Titration bestimmt und daraus der Umsatz berechnet. Diese Prozedur wurde auch mit carboxyliertem Polypox^{®} R20 (Fa. UPPC AG), ein trifunktionelles Epoxid, welches von uns mit CO₂ carboxyliert wurde, 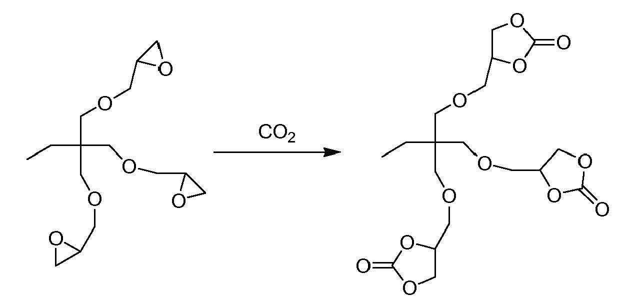 bei Raumtemperatur durchgeführt. Die Ergebnisse sind in Fig. 5 graphisch wiedergegeben und zeigen die hervorragende Reaktivität unseres Bindemittels.

### Beispiel 16: Filmbildung mit Isophorondiamin

0,000666 mol des Bindemittels 3 aus Beispiel 7 bzw. 10 wurden mit 0,001 mol Isophorondiamin gemischt (Bindemittel im Überschuss). Die beiden Komponenten wurden per Hand 20 Sekunden zusammengerührt, danach wurde ein 300 µm dicker Film aufgezogen (Topfzeit 2 min, klebfrei nach 6 Stunden).

### Beispiel 17: Filmbildung mit 1,3-Cyclohexan-bis(methylamin)

0,000666 mol des Bindemittels 3 aus Beispiel 7 bzw. 10 wurden mit 0,001 mol 1,3-Cyclohexan-bis(methylamin) gemischt (Bindemittel im Überschuss). Die beiden Komponenten wurden per Hand 20 Sekunden zusammengerührt, danach wurde ein 300 µm dicker Film aufgezogen (Topfzeit 2 min, klebfrei nach 7 Stunden)

## Patentansprüche

1. 2-Oxo-1,3-dioxolan-4-carbonsäureester der Formel (V), 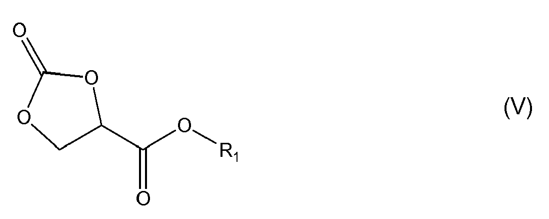 **dadurch gekennzeichnet, dass**
R₁ aus geradkettigen oder verzweigten aliphatischen Gruppen, Arylgruppen, Aralkylgruppen sowie Alkarylgruppen ausgewählt ist.

2. 2-Oxo-1,3-dioxolan-4-carbonsäureester nach Anspruch 1, **dadurch gekennzeichnet, dass** R₁ aus geradkettigen oder verzweigten Alkylgruppen, insbesondere Methyl, Ethyl, n-Propyl, iso-Propyl, n-Butyl, sec-Butyl, tert-Butyl, n-Pentyl, neo-Pentyl, n-Hexyl, 2-Ethyl-n-hexyl, sowie aus Cyclohexyl, Phenyl und Benzyl ausgewählt ist.

3. 2-Oxo-1,3-dioxolan-4-carbonsäureester nach Anspruch 1 oder 2, ausgewählt aus 4-Methoxycarbonyl-2-oxo-1,3-dioxolan und 4-Ethoxycarbonyl-2-oxo-1,3-dioxolan.

4. 2-Oxo-1,3-dioxolan-4-carbonsäureester nach Anspruch 1, **dadurch gekennzeichnet, dass** R₁ ein durch Abstraktion der OH -Gruppen eines n-wertigen Polyols abgeleiteter n-wertiger Rest ist, der mit maximal n-1 weiteren 2-Oxo-1,3-dioxolan-4-carboxylatgruppen der allgemeinen Formel (VI) 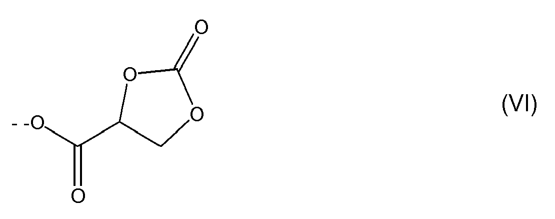 substituiert sein kann.

5. 2-Oxo-1,3-dioxolan-4-carbonsäureester nach Anspruch 4, **dadurch gekennzeichnet, dass** das n-wertige Polyol C₂₋₄-(Poly)Oxyalkylengruppen umfasst.

6. 2-Oxo-1,3-dioxolan-4-carbonsäureester nach Anspruch 4 oder 5, **dadurch gekennzeichnet, dass** n = 2 bis 5 ist.

7. Verfahren zur Herstellung der 2-Oxo-1,3-dioxolan-4-carbonsäureester nach einem der Ansprüche 1 bis 3, **dadurch gekennzeichnet, dass** ein Epoxid der Formel (VII), 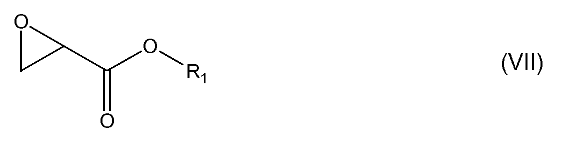 wobei R₁ die angegebene Bedeutung aufweist, mit CO₂ umgesetzt wird.

8. Verfahren nach Anspruch 7, **dadurch gekennzeichnet, dass** die Umsetzung bei einer Temperatur von 15 °C bis 150 °C, vorzugsweise von 30 °C bis 100 °C, und einem Druck von 1 bar bis 75 bar, vorzugsweise von 1 bar bis 30 bar, durchgeführt wird.

9. Verfahren nach Anspruch 7 oder 8, **dadurch gekennzeichnet, dass** die Umsetzung ohne Lösungsmittel oder in einem polaren aprotischen Lösungsmittel durchgeführt wird.

10. Verfahren nach einem der Ansprüche 7 bis 9, **dadurch gekennzeichnet, dass** die Umsetzung in Gegenwart eines Katalysators, ausgewählt aus Metallhalogeniden und Halogensalzen organischer Stickstoffverbindungen sowie Mischungen davon, durchgeführt wird.

11. Verfahren zur Herstellung der 2-Oxo-1,3-dioxolan-4-carbonsäureester nach einem der Ansprüche 4 bis 6, **dadurch gekennzeichnet, dass** ein 2-Oxo-1,3-dioxolan-4-carbonsäureester nach Anspruch 2 oder 3 mit einem n-wertigen Polyol gemäß der Definition eines der Ansprüche 4 bis 6 umgeestert wird.

12. Verfahren nach Anspruch 11, **dadurch gekennzeichnet, dass** die Umesterung in Gegenwart eines enzymatischen Katalysators oder eines sauren Kationenaustauschers durchgeführt wird.

13. Verwendung der 2-Oxo-1,3-dioxolan-4-carbonsäureester nach einem der Ansprüche 1 bis 6 zur Herstellung von Hydroxyurethanen.

14. Verwendung der 2-Oxo-1,3-dioxolan-4-carbonsäureester nach einem der Ansprüche 1 bis 3 als Endgruppen zum Blockieren von Aminen.
